# EUROPÄISCHE PATENTSCHRIFT

(11) **EP 0 292 899 B1**
(45) Veröffentlichungstag und Bekanntmachung des Hinweises auf die Patenterteilung: **14.07.1993**
(21) Anmeldenummer: 88108188.9
(22) Anmeldetag: 21.05.1988
(51) Int. Cl.: C12M 3/00, C12N 15/89

(54) **Verfahren zur Mikroinjektion in Zellen bzw. zum Absaugen aus einzelnen Zellen oder ganzer Zellen aus Zellkulturen**
Process for microinjection into cells, possibly for suction out of isolated cells or entire cells out of cell cultures
Procédé de micro-injection dans des cellules, éventuellement pour aspirer hors de cellules isolées ou de cellules entières de cultures de cellules

(30) Priorität: 29.05.1987 DE 3718066
(43) Veröffentlichungstag der Anmeldung: 30.11.1988
(73) Patentinhaber: Firma Carl Zeiss, D-73446 Oberkochen (DE); CARL ZEISS-STIFTUNG HANDELND ALS CARL ZEISS, D-89518 Heidenheim (Brenz) (DE)
(72) Erfinder: Kettler, Albrecht, Dr.rer.nat., D-7080 Aalen (DE); Nasse, Hubert, Dr.Dipl.-Phys., D-7080 Aalen (DE); Geis, Walter, Dr. Dipl.-Chem., D-7080 Aalen (DE); Wilke, Volker, Dr. Dipl.-Phys., D-7080 Aalen 15 (DE); Ansorge, Wilhelm, Dr., D-6901 Gaiberg (DE)

(56) Entgegenhaltungen:
- GB-A- 2 022 287
- GB-A- 2 154 608
- US-A- 4 662 228

## Beschreibung

Für die biologische Forschung sowie die Gentechnologie spielt das Einbringen von Substanzen in lebende Zellen eine bedeutende Rolle. Hierzu existieren eine Reihe unterschiedlicher Verfahren: so kann z.B. die einzuschleusende Substanz zunächst in das die Zellen umgebende Medium gebracht werden. Damit nun die Substanz in die Zellen gelangen kann, muß die Zellmembran penetriert werden. Dies kann durch ungezielte mechanische, elektrische oder chemische Reize geschehen oder durch einen Laserstrahl, der auf die Zelle fokussiert wird.

Alle diese Verfahren haben den Nachteil, daß die Substanz, die in die Zellen eingebracht werden soll, zum größten Teil im umgebenden Medium verbleibt. Vor allem ist es mit den genannten Methoden nicht möglich Substanzen gezielt nur in den Kern einer Zelle einzubringen. Es wurden daher Methoden entwickelt, bei denen die Substanz mit Hilfe einer Glaskapillare, die an einem Ende zu einer feinen Spitze ausgezogen ist, direkt in einzelne Zellen oder Zellkerne injiziert werden kann.

So ist z.B. aus der Firmenschrift "Arbeitsplatz zur Mikroinjektion in lebende Zellen" der Firma Carl Zeiss, Druckschrift Nr. W41-131-d (XI 86) ein System auf der Basis eines umgekehrten Mikroskopes beschrieben, an dessen Tisch eine schräg zur optischen Achse ausgerichtete Glaskapillare über einen Mikromanipulator befestigt ist. Die Kapillare läßt sich mit Hilfe des Mikromanipulators in drei Achsen gezielt in dem auf dem Tisch des Mikroskops befindlichen Kulturgefäß bewegen. Zur Injektion in die visuell unter dem Mikroskop beobachteten Zellen sind folgende Einzelschritte durchzuführen:
Zuerst muß die Kapillarenspitze grob in das Bildfeld des Objektivs gebracht werden. Anschließend wird die Spitze der Kapillare durch eine Bewegung des Mikromanipulators (x,y) über der interessierenden Zelle positioniert. Schließlich wird durch eine Bewegung in z-Richtung, d.h. parallel zur optischen Achse des Mikroskops die Kapillarenspitze in die Zelle gebracht, der Injektor betätigt und die Kapillare dann, wenn ausreichend Substanz in die Zelle gelangt ist, wieder angehoben. Hierbei erfolgt die Injektion nicht in Achsrichtung der Kapillare.

Das vorbeschriebene, rein manuelle Verfahren, erfordert mehrere, präzis hintereinander durchzuführende Einzelschritte, wobei einige Erfahrung notwendig ist, um die Zellen richtig zu treffen und insbesondere um die Kapillare nicht zu weit abzusenken und damit abzubrechen. Außerdem erfolgt der Einstich in die Zellen nicht in Achsrichtung der Kapillare, so daß die Zellmembran durch den Injektionsvorgang relativ stark verletzt wird.

Aus der DE-OS 35 11 700 ist ein anderes Injektionssystem bekannt, das eine in vertikaler Richtung angeordnete und an den Fokussiertrieb des inversen Mikroskops gekoppelte Kapillare besitzt. Bei diesem System muß die Kapillare in z-Richtung erst einmal so justiert werden, daß eine Bewegung der Kapillare innerhalb des Tiefenschärfenbereiches des Mikroskops zu einem Einstechen in die Zelle führt. Anschließend wird die Zelle, in die injiziert werden soll, mit Hilfe des Objektführers unter die nur ungenau erkennbare Kapillarenspitze gefahren. Danach wird der eigentliche Injektionsvorgang durchgeführt, indem die Kapillare mit Hilfe des Fokussiertriebs abgesenkt wird, wobei sie in Achsrichtung in die Zelle eindringt; nach erfolgter Injektion wird die Kapillare wieder angehoben.

Bei diesem System ist die Gefahr, daß die Kapillarenspitze abbricht oder zumindest verstopft, wenn sie auf den Gefäßboden gelangt, besonders hoch, da die Kapillarenspitze senkrecht auf den Gefäßboden auftrifft. Außerdem verliert man sehr leicht den Überblick darüber, in welche Zellen bereits injiziert wurde, da der Tisch des Mikroskops bei jeder Injektion verschoben wird, wodurch sich das Bildfeld dauernd ändert.
All dies hat zur Folge, daß mit den bekannten manuellen Systemen zur Zellinjektion selbst von geübtem Personal pro Stunde nicht mehr als etwa 300 Zellen injiziert werden können.

Weiterhin sind mikrobiologische Verfahren bekannt, die ein Absaugen von Flüssigkeit aus Zellen oder ein Absaugen ganzer Zellen aus Zellkulturen vorsehen. Auch hierbei wird mit einer Kapillare in die Zelle eingestochen bzw. die Kapillare auf der abzusaugenden Zelle positioniert. Bei diesem Verfahren laufen im Prinzip die gleichen Arbeitsschritte ab wie sie vorstehend am Beispiel der Injektion geschildert wurden, und es liegen die gleichen Beschränkungen hinsichtlich der Arbeitsgeschwindigkeit vor.

Es ist die Aufgabe der vorliegenden Erfindung ein System zur Injektion in Zellen bzw. zum Absaugen aus einzelnen Zellen oder ganzer Zellen aus Zellkulturen zu schaffen, das ein schnelleres und sichereres Arbeiten als die bekannten Systeme erlaubt.

Diese Aufgabe wird durch ein Verfahren mit den im Kennzeichen des Anspruchs 1 angegebenen Merkmalen dadurch gelöst, daß
- dem Fernsehbild eine im Bild bewegbare Marke überlagert wird,
- die Marke auf die zu injizierenden oder abzusaugenden Zellen im Fernsehbild positioniert wird und die Bildkoordinaten der markierten Zellen in einem Rechner abgespeichert werden,
- die Bildkoordinaten vom Rechner in Objektkoordinaten des Positioniersystems für die Kapillare bzw. den Zellträger umgerechnet werden
- und anschließend in die ausgewählten Zellen injiziert bzw. aus diesen abgesaugt wird, indem die Kapillare relativ zur jeweiligen Zelle rechnergesteuert auf die berechneten Objektkoordinaten positioniert wird.

Bei dem erfindungsgemäßen Verfahren steuert ein Rechner die Positionierung und das Einstechen der Kapillare in die Zelle.

Da dieser kritische Bewegungsvorgang nunmehr automatisch erfolgt, läßt sich eine höhere Arbeitsgeschwindigkeit erzielen und ist die Gefahr, daß die Kapillare durch Fehlbedienung abbricht, stark vermindert. Der Bediener muß nur noch die zu injizierenden oder abzusaugenden Zellen im Monitorbild markieren. In einer vorteilhaften Weiterbildung ist es sogar möglich, diesen Vorgang durch ein Bildanalysesystem ausführen zu lassen, das bestimmte Zelltypen oder Zellen in einem bestimmten Entwicklungszustand an ihrer geometrischen Form selbstätig erkennt.

Das Verfahren nach der Erfindung stellt eine gleichbleibende Tiefe der Einstichbewegung in die Zelle sicher und liefert daher in Bezug auf die Art und Größe der Zellverletzung reproduzierbarere Ergebnisse als das bisherige, manuell gesteuerte Verfahren. Außerdem ist die Verbleibdauer der Kapillare in der Zelle und damit das injizierte oder abgesaugte Volumen sehr genau vorherbestimmbar und reproduzierbar.

Mit Hilfe des Rechners ist es weiterhin möglich, die Koordinaten aller Zellen, die bereits bearbeitet wurden, abzuspeichern. Dadurch kann sich der Benutzer jederzeit darüber informieren, welche und wieviele Zellen er bereits behandelt hat. Es ist dann auch möglich, jederzeit den Vorgang zu unterbrechen und zu einem späteren Zeitpunkt exakt an derselben Stelle fortzuführen. Weiterhin kann z.B. die Wirkung der Injektion durch Vergleich der Entwicklung der Zellen, in die injiziert, und der Zellen, in die nicht injiziert wurde, über einen langeren Zeitraum kontrolliert werden. Diese Möglichkeit eröffnet sich in vorteilhafter Weise dadurch, daß sich die Zellen in einem Gefäß befinden, das mit Referenzmarken versehen ist und die Koordinaten der injizierten Zellen zusammen mit denen der Referenzmarken gespeichert werden, so daß ein sicheres Wiederauffinden individueller Zellen auch nach einem Wechsel des Probengefäßes gewährleistet ist.

Fur die Einstichbewegung der Kapillare in achsialer Richtung kann ein eigener Antrieb vorgesehen werden. Da der Rechner mehrere Bewegungen miteinander in einer Weise koordiniert, wie es bei manueller Steuerung kaum möglich ist, läßt sich jedoch diese Einstichbewegung in Kapillarrichtung auch durchführen, ohne daß ein separater Antrieb in Achsrichtung der Kapillare vorgesehen werden muß. Die Rechnersteuerung ist in der Lage den gewünschten Bewegungsvorgang durch eine Überlagerung der Verschiebung des Mikroskoptisches und der z-Absenkung des Manipulators zusammensetzen.

Weiterhin ist es vorteilhaft, wenn der gesamte Probenbereich in mehrere aneinander anschließende Felder aufgeteilt ist, die von der Rechnersteuerung nacheinander angefahren werden. Die mit einer Identifikationskennung versehenen zugehörigen Bildfelder können dann vom Benutzer systematisch nacheinander abgearbeitet werden. Mehrfachinjektionen oder ein Vergessen von Zellen sind auf diese Weise sicher vermieden.

Weitere Vorteile der Erfindung ergeben sich aus den Unteransprüchen und werden nachstehend anhand der Figuren der beigefügten Zeichnungen näher erläutert, in denen ein Ausführungsbeispiel für eine Vorrichtung zur Durchführung des erfindungsgemäßen Verfahrens beschrieben ist.
- Fig. 1: ist ein Blockschema, das die Verbindung der einzelnen Komponenten des Arbeitsplatzes darstellt;
- Fig. 2: ist eine perspektivische Skizze der beweglichen Teile des Injektionssystems am Mikroskop (1) aus Fig. 1;
- Fig. 3: zeigt das Bild des Monitors (10) aus Fig. 1;
- in Fig.4: ist das Probengefäß (18) aus Fig. 2 vergrößert dargestellt.

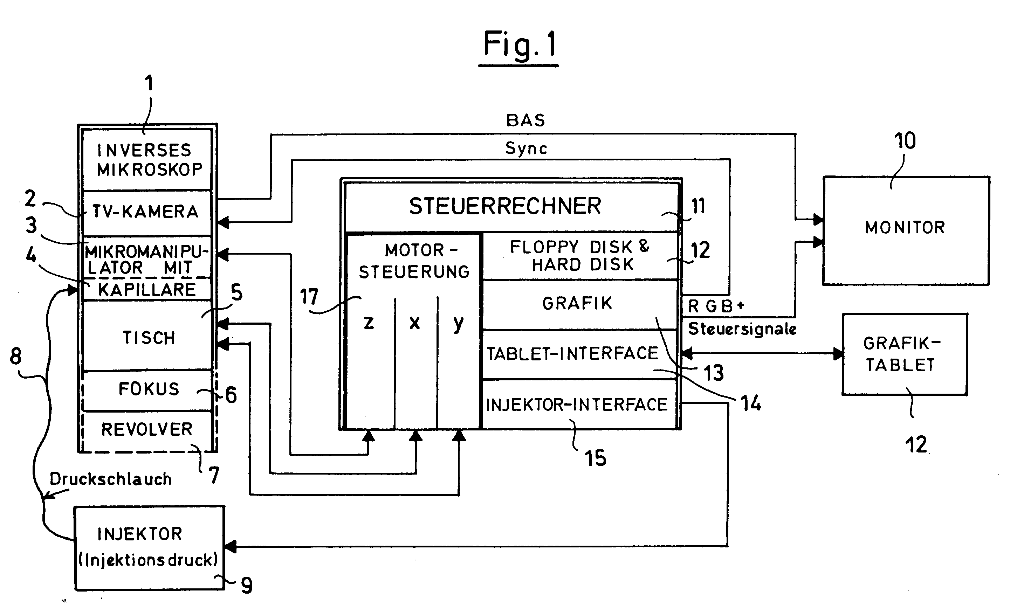

Das im Blockschema nach Fig. 1 dargestellte System zur Zellinjektion baut auf einem inversen Mikroskop (1) auf, das zu diesem Zwecke einen Kondensor mit langer Brennweite besitzt und mit wechselbaren, hier nicht dargestellten Einrichtungen für verschiedene optische Kontrastierungsverfahren wie Phasenkontrast und Differential-Interferenzkontrast versehen ist. Das Mikroskop (1) besitzt einen Revolver (7) für Objektive mit unterschiedlichem Abbildungsmaßstab.

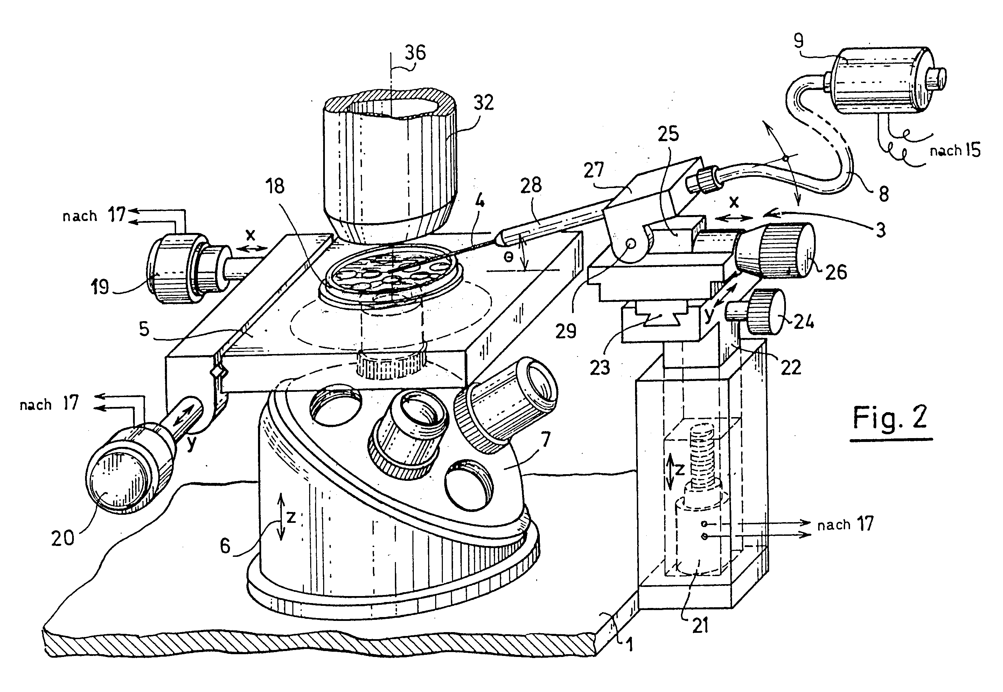

Über dem Objektivrevolver (7) befindet sich ein in zwei Koordinaten (x,y) motorisch bewegbarer Scanningtisch, der üblicherweise eine Schrittauflösung von 0,25 um besitzt, und mit wählbarer Geschwindigkeit bewegt werden kann. Wie aus der perspektivischen Darstellung nach Fig. 2 hervorgeht, trägt der Tisch (5) einen Halter für das Probengefäß (18) mit den zu injizierenden Zellen.

Am Mikroskop (1) ist weiterhin eine TV-Kamera (2) und der mit (3) bezeichnete Mikromanipulator zur Halterung der Injektionskapillare (4) befestigt. Mit Hilfe dieser Fernsehkamera läßt sich ein vergrößertes Bild der zu behandelnden Zellkulturen auf einen Monitor (10) sichtbar machen.

Die Kapillare (4) ist über einen Druckschlauch (8) mit einem Injektor (9) verbunden, der das Ausströmen der Injektionsflüssigkeit aus der Kapillare bewirkt und eine Einstellung von Haltedruck, Injektionsdruck und Spüldruck ermöglicht.

Die vorstehend genannten Komponenten sind auch bei den eingangs zum Stand der Technik genannten, bekannten Systemen zur Mikroinjektion vorhanden und werden deshalb an dieser Stelle nicht mehr detailliert dargestellt.

Wie bereits ausgeführt ist der Tisch (5) des Mikroskops motorisch bewegbar. Die beiden Antriebe für die Tischbewegung in x-und in y-Richtung sind in der detaillierteren Darstellung nach Fig. 2 mit (19 bzw. 20) bezeichnet. Der Mikromanipulator (3) läßt sich ebenfalls motorisch über einen Antrieb (21) verstellen und zwar in z-Richtung, außerdem besitzt der Halter (27) des Mikromanipulators, an dem die Kapillare (4) befestigt ist, Verstellmöglichkeiten für eine Bewegung in x- und y-Richtung. Im beschriebenen Ausführungsbeispiel sind hier manuell zu betätigende Einstellknöpfe (24 und 26) vorgesehen, es ist jedoch auch möglich für diese Bewegungen motorische Antriebe einzusetzen.

Die Kapillare (4) taucht schräg in das Probengefäß (18) auf den Tisch (5) des Mikroskops (1) ein, wobei sich der Neigungswinkel der Kapillare zur Tischebene durch ein Verschwenken des Halters (27) der Kapillare um die Achse (29) einstellen läßt.

Auch der in Fig. 2 dargestellte Aufbau des Positioniersystems für die Kapillare (4) bzw. das Probengefäß (18) ist in seinen Bestandteilen an sich bekannt.

Gemäß der Erfindung sind die Antriebe (19 und 20) des Scanningtisches (5) und der Antrieb (21) für die z-Bewegung des Mikromanipulators an die Motorsteuerung des in Fig. 1 mit (11) bezeichneten Rechnes angeschlossen. Bei diesem Rechner handelt es sich z.B. um einen graphikfähigen Personal-Computer wie den IBM PC/XT. Die Graphikkarte (13) des Rechners (11) ist ebenfalls an den Monitor (10) angeschlossen. Außerdem ist die Fernsehkamera (2) am Mikroskop (1) extern durch die Synchronimpulse der Graphikkarte synchronisiert. Der Monitor (10) enthält eine Mischstufe, in der die von der Graphikkarte (13) erzeugten Symbole dem Videosignal der Fernsehkamera (2) überlagert werden.

An den Rechner (11) ist weiterhin ein Graphiktablett (12) angeschlossen, mit dessen Hilfe der Benutzer, wie nachstehend noch beschrieben wird, die von der Graphikkarte erzeugten Marken auf dem Bild des Monitors (10) verschieben kann. Natürlich kann anstelle des Graphiktabletts auch eine Maus, eine sogenannte Rollkugel oder ein Joystick zur Steuerung der Bewegung der Marken verwendet werden.

Der Steuerrechner (11) ist weiterhin über ein Injektor-Interface (15) mit der Injektionseinheit (9) für die Kapillare (4) verbunden und steuert u.a. die Injektionszeit und damit die Menge der in die Zellen zu injizierenden Flüssigkeit. Über das Interface (15) läßt sich auch der Injektionsdruck zeitabhängig entsprechend dem Bewegungsvorgang steuern, den die Kapillare (4) ausführt. Beispielsweise wird vor der Injektion ein geringer Überdruck eingestellt, um ein Zurückströmen von Flüssigkeit in die Kapillare zu verhindern, während der Einstichbewegung wird der Injektionsdruck erhöht, um ein Verstopfen der Kapillare zu vermeiden, und anschließend wird der eigentliche Injektionsdruck eingestellt.

Die Mikroinjektion mit Hilfe der vorstehend beschriebenen Vorrichtung läuft nun folgendermaßen ab:

### Vorarbeiten

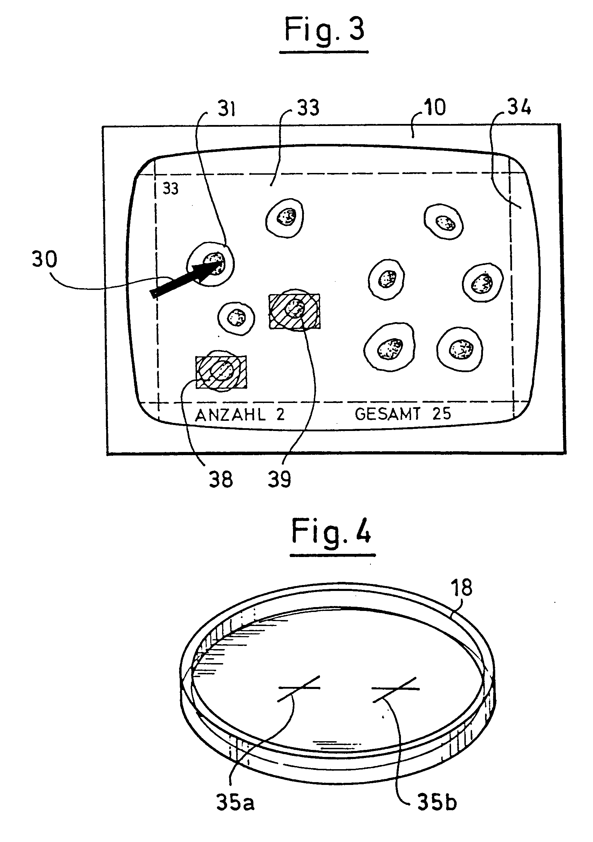

Das Zellgefäß (18) wird auf dem Mikroskoptisch (5) befestigt. Am Boden des in Fig. 4 beispielhaft dargestellten Gefäßes sind zwei Referenzmarken (35a, 35b) angebracht. Mit Hilfe dieser Referenzmarken wird das Gefäß (18) so ausgerichtet, daß diese etwa in x-Richtung des Tisches liegen. Die Referenzmarken definieren außerdem den Ursprung des Bildkoordinatensystems in eindeutiger Weise und erlauben ein Wiederauffinden der im Gefäß (18) befindlichen Zellen. Außerdem kann es vorteilhaft sein den Boden des Zellgefäßes (18) in einem regelmäßigen Muster zu ritzen um aneinandergrenzende Felder zu markieren.

Anschließend wird die Kapillare mit Injektionsflüssigkeit gefüllt, in den Kapillarenhalter (27) eingespannt und durch manuelle x-, y-Verstellung mit Hilfe der Drehknöpfe (24) und (26) unter dem Kondensor (32) etwa auf der Achse (36) des Mikroskops in das Bildfeld gebracht.

Falls die Steuerung des Druckes nicht durch den Rechner erfolgt, wird der gewünschte Injektions- bzw. Haltedruck am Injektor (9) eingestellt.

### Dateneingabe

Anschließend wird im Rechner (11) das zur Steuerung des Arbeitsablaufes verwendete Programm aufgerufen. Dieses Programm verlangt die Eingabe einiger für die Programmausführung notwendiger Parameter: Den Abbildungsmaß des verwendeten Objektivs, den Injektionswinkel 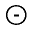 , unter dem die Kapillare (4) gegen die Tischoberfläche geneigt ist. Außerdem ist es möglich eine Anzahl von Feldern zu wählen, die aneinander angrenzen und die der motorgetriebene Tisch (5) unter Rechnersteuerung nacheinander anfährt. Die zugehörigen Bildfelder (33,34) werden dann wie in Fig. 3 dargestellt mit einer von der Graphikkarte (13) erzeugten Umrandung und mit einer Identifikationskennziffer (33) versehen auf dem Monitor (10) dargestellt. An dieser Stelle kann auch der Injektionsmodus gewählt werden wie z.B. dauerndes Ausströmen der Injektionsflüssigkeit aus der Kapillare oder Ausströmen nur nach dem Einstich der Kapillare in eine Zelle, bzw. der gewünschte Druckverlauf angegeben werden. Außerdem ist es möglich die Geschwindigkeit der Einstichbewegung der Kapillare auszuwählen. Beispielsweise wird für Zellen mit nachgiebiger Zellmembran eine höhere Geschwindigkeit benötigt als für Zellen mit relativ harter Zellmembran.

### Gefäßposition

Nach der Dateneingabe, die menügesteuert unter Verwendung des Graphiktabletts (12) und des Bildschirms des Monitors (10) erfolgt, wird auf dem Bildschirm (10) das vom Mikroskop erzeugte Bild aufgerufen, auf den Gefäßboden fokussiert und die beiden Referenzmarken auf dem Gefäßboden angefahren. Hierbei dient das Graphiktablett zur Steuerung der Tischposition über dem Mikroskopobjektiv.

Die Referenzmarken auf dem Gefäßboden werden anschließend mit dem in Fig. 3 dargestellten pfeilförmigen Cursor (30) markiert und dabei die Positionen der Gefäßmarken mit dem Koordinatensystem des Rechners in Übereinstimmung gebracht. Wenn der Rechner die etwaigen Positionen der Markierungen bereits kennt, was z.B. bei Verwendung st ndardisierter Gefäße, oder bei Fortsetzung einer unterbrochenen Injektion ohne Abnahme des Gefäßes vom Tisch der Fall ist, so kann er die Markierungen selbst ins Bildfeld der Fernsehkamera fahren und der Bediener braucht nur noch die exakte Position der Marken am Gefäßboden mit dem Cursor (30) markieren.

### Kapillare justieren / Testinjektion

Nach korrekter Platzierung des Gefäßes wird mit dem Fokussiertrieb auf die Kapillarenspitze fokussiert und diese mit Hilfe der x/y-Verstelleinheit (24, 26) des Mikromanipulators (3) etwa in die Mitte des Gesichtsfelds des Mikroskops gebracht. Anschließend wird auf die Ebene der Zellen am Gefäßboden fokussiert.

Die Kapillare wird dann interaktiv unter Steuerung über das Graphiktablett (12) soweit abgesenkt, bis die Spitze der Kapillare (4) in eine der Zellen eindringt. Diese Position wird dem Rechner übergeben, so daß dieser nun eine Information über das Ausmaß der z-Bewegung beim späteren, automatischen Injizieren besitzt. Die Position der Kapillarenspitze in der xy-Ebene im Bildkoordinatensystem wird dem Rechner dadurch mitgeteilt, daß der Cursor (30) mit seiner Spitze auf die Kapillarenspitze positioniert wird.

Auf dem Bildschirm wird nun eine Zelle ausgewählt, in die eine Testinjektion ausgeführt werden soll, indem diese Zelle ebenfalls mit dem Cursor (30) markiert wird. Nach dem Markieren fährt der Rechner die Kapillarenspitze automatisch in die markierte Position und sticht dabei die Zelle an. Falls die Kapillare dabei nicht weit genug in die Zelle eindringt, wird sie nochmals abgesenkt, bis die Testinjektion erfolgreich ist. Hierbei findet eine Korrektur der während des Justiervorganges ermittelten Werte für die z-Absenkung statt.

Im Zuge dieser Testinjektion können die vom Rechner einzuhaltende Verweilzeit der Kapillare in der Zelle sowie der Injektionsdruck geprüft und gegebenenfalls modifiziert werden. Verläuft die Testinjektion erfolgreich, so kann mit dem eigentlichen, routinemäßigen Injizieren begonnen werden.

### Zellen eines Bildfelds markieren

Hierzu wird mit Hilfe des Graphiktabletts (12) die Bildschirm-Cursor-Marke (30) nacheinander auf den ausgewählten Zellen positioniert, in die injiziert werden soll. Deren Koordinaten im Bildschirmkoordinatensystem werden durch Drücken einer Taste jeweils an den Rechner übergeben und dort gespeichert. Der Rechner verwaltet die Koordinaten der markierten Zellen und zeigt ständig die Anzahl der im Bildfeld markierten Zellen sowie der insgesamt markierten Zellen an. Außerdem wird dem Bild jeder markierten Zelle ein rechteckförmiges Markierfeld (38 bzw. 39) in Fig. 3 überlagert, wodurch die markierten Zellen gekennzeichnet sind.

Es ist auch möglich die Koordinaten der ausgewählten Zellen dadurch zu erfassen, daß diese durch Bewegen des Tisches (5) nacheinander unter die, beispielsweise in der Bildmitte fixierte Cursor-Marke (30) gebracht werden. Durch Drücken einer Taste werden dann die Tischkoordinaten an den Rechner übergeben.

Ist eine Markierung irrtümlich gesetzt worden, so ist es möglich die gesetzte Markierung wieder zu löschen, wodurch verhindert wird, daß die Zelle beim nachfolgenden, automatischen Injizieren von der Kapillare angefahren wird.

### Injektion der markierten Zellen

Der Injektionsvorgang wird vom Rechner gesteuert, kann jedoch jederzeit vom Bediener unterbrochen werden, z.B. falls die Kapillare verstopft ist. Im Zuge des automatischen Injektionsvorganges wandelt der Rechner die Bildfeldkoordinaten der markierten Zellen in die zur Steuerung des Tisches (5) und des z-Antriebs (21) des Mikromanipulators (3) nötigen Koordinatenwerte des Handhabungssystems um. Anschließend verfährt er die Kapillarenspitze relativ zu den markierten Zellen und positioniert die Kapillarenspitze in den Zellen, indem er sie durch eine entsprechend dem eingegebenen Winkel für die Neigung der Kapillare zusammengesetzten Bewegung mit der Spitze der Kapillare ansticht, während der voreingestellten Zeit das Ventil des Injektors (9) öffnet, die Kapillare zurückzieht und zur nächsten markierten Zelle verfährt.

Es ist auch möglich das Anstechen der Zellen durch einen Antrieb zu bewirken, der die Kapillare (4) achsial bewegt. Ein solcher Antrieb kann aus einem im Kapillarenhalter (27) angeordneten Motor bestehen, der vom Rechner (11) angesteuert wird, sobald die Spitze der Kapillare (4) eine vorgegebene Position bezüglich der zu behandelnden Zelle erreicht hat.

### Aufsuchen des nächsten Bildfelds

Wurde zu Beginn definiert, wieviel Bildfelder in x- und y-Richtung bearbeitet werden sollen, so wird das nächste Bildfeld, das sich unmittelbar an die vorausgegangenen anschließt, automatisch angefahren. Liegt keine Definition vor, oder sind bereits alle definierten Felder bearbeitet, dann wird das nächste Bildfeld vom Bediener gewählt. Hierzu kann der Tisch frei oder um jeweils genau ein Bildfeld in x- oder y-Richtung verfahren werden. Die momentane Position innerhalb des Bildfeldrasters wird dabei durch eine Identifikationskennziffer (33) angezeigt. Gelangt man beim Verfahren des Tisches in ein Bildfeld, in dem bereits Zellen injiziert wurden, so werden diese ebenfalls auf dem Monitorbild mit einem Markierfeld versehen, um Mehrfach-Injektionen sicher zu vermeiden.

Die letzten drei Teilschnitte:
1. Markieren der Zellen mit dem Cursor durch den Benutzer,
2. Injizieren in die markierten Zellen unter Rechnersteuerung, und
3. Automatisches Anfahren des nächsten Feldes
werden solange wiederholt, bis die gewünschte Anzahl von Zellen injiziert ist bzw. alle definierten Bildfelder nacheinander abgearbeitet sind.

Wenn die Injektion aus irgendeinem Grunde vorzeitig abgebrochen werden muß, so kann diese zu einem beliebigen Zeitpunkt an der richtigen Stelle fortgesetzt werden, auch wenn das Zellgefäß zwischendurch aus der Halterung auf dem Mikroskoptisch (5) entnommen wurde. Es ist dann nur erforderlich, das Bildkoordinatensystem nochmals wie unter "Gefäßposition" beschrieben an die Referenzmarken am Gefäßboden anzubinden.

Da die Zellkoordinaten wahlweise auch permanent gespeichert werden, kann man zu einem späteren Zeitpunkt alle Felder gezielt nochmals anfahren, in denen Zellen injiziert wurden. Man hat so die Möglichkeit die Entwicklung dieser Zellen im Vergleich zu denen, die nicht injiziert wurden, zu verfolgen.

### Geometrische Kalibrierung

Eine geometrische Kalibrierung ist nur bei der ersten Inbetriebnahme der Vorrichtung notwendig. Sie dient dazu, um aus der Position einer Zelle auf dem Monitor ihre tatsächliche Position auf dem Mikroskoptisch zu errechnen. Da in die Umrechnung die Abbildungsmaßstäbe aller verwendeten Wechselobjektive eingehen, müssen die Monitorkoordinaten für jedes Objektiv in Einheiten der Tischkoordinaten geeicht werden.

Hierzu wird ein gut erkennbares Objekt durch Bewegen des Mikroskoptisches auf dem Monitor (10) in x- und y-Richtung verschoben. Die jeweilige Monitorposition wird dem Rechner durch Markieren des Objekts auf dem Bildschirm des Monitors (10) mit der Spitze der Cursormarke (30) mitgeteilt. Aus den zugehörigen Koordinaten- und Monitorpositionen ermittelt der Rechner die Eichfaktoren in x- und y-Richtung. Diese den einzelnen Objektiven zugeordneten Eichfaktoren werden im Rechner (11) permanent gespeichert.

### Weitere Ausbaustufen der Vorrichtung

Wie unter der Überschrift "Dateneingabe" beschrieben wird vor der eigentlichen Injektion dem Rechner das verwendete Objektiv mitgeteilt. Dieser Vorgang läßt sich zusätzlich vereinfachen, wenn ein motorisierter Objektivrevolver verwendet ist und den Revolverstellungen bzw. den damit verbundenen Objektiven eine vom Rechner lesbare Codierung zugeordnet ist. Es entfällt dann die manuelle Eingabe des verwendeten Objektivs durch den Benutzer.

In der bisher beschriebenen Vorrichtung erfolgt das Markieren der Zellen eines Bildfeldes manuell, indem die Marke unter visueller Betrachtung durch den Benutzer auf die interessierenden Zellen aufgesetzt wird. Dieser Vorgang läßt sich dadurch automatisieren, daß die Auswahl der interessierenden Zellen und die Ermittlung der Zellkoordinaten beispielsweise durch Schwerpunktbildung mit Hilfe eines Bildanalysesystems erfolgt.

Außerdem ist die beschriebene Vorrichtung nicht nur für die Injektion von Flüssigkeit in Zellen, sondern auch für das Absaugen von Flüssigkeit aus den Zellen bzw. ganzer Zellen aus einer Zellkultur geeignet.

Schließlich ist der erste Teil des Verfahrens bis zur Markierung interessierender Zellen und der Speicherung ihrer Koordinaten relativ zu den Referenzmarken auf dem Gefäßboden auch dazu geeignet, lebende Zellen "virtuell" zu markieren, deren Entwicklung (Wachstum, Bewegung, morphologische Veränderungen etc.) über einen längeren Zeitraum interaktiv verfolgt werden soll, ohne daß in die Zellen injiziert bzw. abgesaugt wird.

Mit der anhand der Figuren beschriebenen Vorrichtung zur rechnerunterstützten Mikroinjektion lassen sich bedeutend mehr Zellen pro Zeiteinheit injizieren, als mit denen eingangs zum Stand der Technik genannten Systemen. Es hat sich erwiesen, daß es durchaus möglich ist 1500 Zellen pro Stunden zu injizieren, was einer Beschleunigung der bekannten Verfahren um einen Faktor 5 bedeutet. Dies ist nicht nur durch die Möglichkeit des schnellen Positionierens einer Cursormarke auf einem Bildschirm im Vergleich zur feinfühligen Positionierung der Kapillare selbst im Gefäß bedingt, sondern auch dem Umstand zu verdanken, daß es aufgrund der sicheren Rechnersteuerung der Einstechbewegung und des Injektionsdrucks sehr viel seltener zu einem Bruch der Kapillare und damit verbunden zu einem zeitaufwendigen Auswechseln und Neujustieren kommt.

## Patentansprüche

1. Verfahren zur Mikroinjektion in Zellen bzw. zum Absaugen aus einzelnen Zellen oder ganzer Zellen aus Zellkulturen, wobei relativ zu den unter einem Mikroskop (1) mit angeschlossener Fernsehkamera (2) beobachteten Zellen eine Kapillare (4) positioniert wird, dadurch gekennzeichnet, daß
- dem Fernsehbild eine Marke (30) überlagert wird,
- die Marke (30) durch Relativbewegung zwischen Bild (10) und Marke (30) auf die zu injizierenden Zellen (31) im Fernsehbild positioniert wird und die Bildkoordinaten der markierten Zellen in einem Rechner (11) abgespeichert werden,
- die Bildkoordinaten vom Rechner in Objektkoordinaten des Positioniersystems (19, 20, 21) für die Kapillare (4) bzw. den Zellträger (Tisch 5) umgerechnet werden
- und anschließend in die ausgewählten Zellen (31) injiziert bzw. aus diesen abgesaugt wird, indem die Kapillare (4) relativ zur jeweiligen Zelle (31) rechnergesteuert auf die berechneten Objektkoordinaten positioniert wird.

2. Verfahren nach Anspruch 1, dadurch gekennzeichnet, daß die Einstichbewegung der Kapillare (4) in Kapillarrichtung durch eine überlagerte Bewegung des Positioniersystems (19 - 21) für die Kapillare (4) bzw. den Zellträger (Tisch 5) in zwei Koordinaten entsprechend dem Neigungswinkel 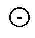 der Kapillare (4) erfolgt.

3. Verfahren nach Anspruch 1, dadurch gekennzeichnet, daß der Probenbereich in mehrere Felder (33, 34) fester Größe aufgeteilt ist, die aneinander anschließen und dem Fernsehbild die Grenzen der Bildfelder sowie eine Identifikationskennung für das jeweilige Bildfeld überlagert werden.

4. Verfahren nach Anspruch 1, dadurch gekennzeichnet, daß sich die Zellen in einem Gefäß (18) mit Referenzmarken (35) befinden und das Bildkoordinatensystem in einem vorab durchgeführten Kalibriervorgang mit den im Fernsehbild sichtbaren Referenzmarken in Übereinstimmung gebracht wird.

5. Verfahren nach Anspruch 1, dadurch gekennzeichnet, daß nach dem Injektions- oder Absaugvorgang die Koordinaten der bearbeiteten Zellen (31) zusammen mit einer Identifikationskennziffer sowie die Koordinaten der Referenzmarke (35) permanent gespeichert werden.

6. Verfahren nach Anspruch 1, dadurch gekennzeichnet, daß vor der rechnergesteuerten Injektion bzw. Absaugvorgang manuell ein Testvorgang durchgeführt wird, wobei die Koordinaten der Kapillarspitze (40) und der Zelle (31) in Übereinstimmung gebracht und vom Rechner übernommen werden.

7. Verfahren nach Anspruch 1, dadurch gekennzeichnet, daß die Steuerung der Injektionszeit bzw. Injektionsmenge oder Absaugmenge durch den Rechner (11) entsprechend vorgegebener Werte erfolgt.

8. Verfahren nach Anspruch 1, dadurch gekennzeichnet, daß die Geschwindigkeit der Einstichbewegung der Kapillare (4) in die Zelle (31) wählbar ist.

9. Verfahren nach Anspruch 1, dadurch gekennzeichnet, daß der zeitliche Verlauf des Injektionsdruckes vom Rechner (11) entsprechend dem Bewegungsvorgang der Kapillare (4) gesteuert wird.

10. Verfahren nach Anspruch 1, dadurch gekennzeichnet, daß die zu injizierenden oder abzusaugenden Zellen durch ein Bildanalysesystem ausgewählt werden.

11. Vorrichtung zur Durchführung des Verfahrens nach einem der Ansprüche 1 bis 10, gekennzeichnet durch
- ein Mikroskop (1) mit einem in mindestens zwei Richtungen positionierbaren Objekttisch (5),
- einer Fernseheinrichtung (2) zur Darstellung des mikroskopischen Bildes auf einem Monitor (10),
- einen graphikfähigem Rechner (11) mit angeschlossenem Speicher (12) und einer Einrichtung (13) zur Überlagerung der Graphiksysmbole auf dem Monitor (10),
- eine Einrichtung (14) zur Positionierung einer Graphikmarke auf dem Monitor (10),
- einen Mikromanipulator (3) mit daran befestigter Injektionseinheit (4, 28) bzw. Saugeinheit, der in mindestens einer Richtung vom Rechner (11) positionierbar ist,
- Probengefäße (18) zur Aufnahme der zu injizierenden bzw. abzusaugenden Zellen (31)
- sowie ein Rechenprogramm zur Umrechnung der über die Graphikeinrichtung (13 - 14) bestimmten Bildkoordinaten in Objektkoordinaten und zur Steuerung des Mikromanipulators (3) bzw. des Objekttisches (5) durch den Rechner (11).

12. Vorrichtung nach Anspruch 11, dadurch gekennzeichnet, daß die Probengefäße (18) mit einer sichtbaren Markierung (35) versehen sind, die zur Lageerkennung des Gefäßes auf dem Mikroskoptisch dient.

13. Vorrichtung nach Anspruch 11, dadurch gekennzeichnet, daß die Kapillare (4) der Injektionseinheit bezüglich ihrer Winkellage 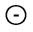 relativ zur optischen Achse (36) des Mikroskops (1) einstellbar ist.

14. Vorrichtung nach Anspruch 11, dadurch gekennzeichnet, daß die Injektionseinheit einen Antrieb für eine achsiale Bewegung der Kapillare (4) besitzt.

15. Vorrichtung nach Anspruch 11, dadurch gekennzeichnet, daß der Rechner (11) mit den Steuerungen (16) für die Antriebe des Objekttisches (5) bzw. des Mikromanipulators (3) verbunden ist.

16. Vorrichtung nach Anspruch 11, dadurch gekennzeichnet, daß das Rechenprogramm eine Speicherung mehrerer über die Graphikeinrichtung bestimmter Bildkoordinatensätze bzw. daraus berechneter Objektkoordinatensätze vorsieht.

## Claims

1. Method for microinjection into cells or for drawing off by suction from individual cells or for drawing off by suction of entire cells from cell cultures wherein a capillary (4) is positioned relative to the cells observed by a microscope (1) to which a camera (2) is connected, characterized by the steps of
- superposing a mark (30) on the television image,
- positioning the mark (30) on the cells selected for injection by a relative movement between the television image (10) and the mark (30) and storing the image coordinates of the marked cells (31) within a computer (11),
- converting the image coordinates into object coordinates of the positioning system (19, 20, 21) of the capillary (4) or the cell-carrier (stage 5) by the computer
- and subsequently injecting into the selected cells (31) or drawing off from them by suction, whereby the capillary (4) is positioned relative to the respective cell (31) onto the calculated object coordinate by computer controlled positioning.

2. Method according to claim 1, characterized by the fact that the injection movement of the capillary (4) in the direction of the capillary is performed by superposed movement of the positioning system (19-21) of the capillary (4) or the cell carrier (stage 5) in two coordinate directions in correspondence to the angle of inclination Θ of the capillary (4).

3. Method according to claim 1, characterized by the fact that the specimen region is subdivided into several mutually adjoining fields (33, 34) of fixed size and that the boundaries of the image fields as well as an identification character for each respective image field is superposed upon the television image.

4. Method according to claim 1, characterized by the fact that the cells are disposed in a vessel (18) with reference marks (35) which are visible in the television image and that the image coordinate system is brought into correspondence with the reference marks by a prior calibration operation.

5. Method according to claim 1, characterized by the fact that the coordinates of the treated cells (31) together with an identification number and the coordinates of the reference marks (35) ar stored permanently after the injection or drawing off by suction process.

6. Method according to claim 1, characterized by the fact that a manual test operation is carried out in advance of the computer-controlled injection or drawing off by suction process, whereby the coordinates of the capillary tip (40) and the cell (31) are brought into correspondence with each other and taken over by the computer.

7. Method according to claim 1, characterized by the fact that the control of the duration of injection, quantity of injection or quantity drawn off by suction is performed by the computer (11) in correspondence to predetermined values.

8. Method according to claim 1, characterized by the fact that the speed of the penetration movement of the capillary (4) into the cell (31) is selectable.

9. Method according to claim 1, characterized by the fact that the temporal dependency of the injection pressure is controlled by the computer (11) in correspondence to the movement of the capillary (4).

10. Method according to claim 1, characterized by the fact that the cells selected for injection or drawing off by suction are selected by an image analysing system.

11. Apparatus for carrying out the method according to one of the claims 1-10, characterized by
- a microscope (1) with an object stage (5) which is positionable at least into two directions,
- a television arrangement (2) for showing the microscope image on the television monitor (10)
- a computer (11) having graphics capability with a memory (12) connected to the computer (11) and with means (13) for superposing graphic symbols on the monitor (10)
- means (14) for positioning a graphic mark on the monitor (10),
- a micromanipulator (3) which is positionable into at least one direction by control of the computer (11) and with an injection unit (4, 28) or an unit for drawing off by suction fixed to the micromanipulator (3),
- a specimen vessel (18) for carrying the cells (31) selected for injection or drawing off by suction and
- a computer program for converting image coordinates determined by the graphic means (13, 14) into object coordinates and for controlling the micromanipulator (3) or the object stage (5) via the computer (11).

12. Apparatus according to claim 11, characterized by the fact that viewable markings are provided at the specimen vessel (18) which serve to detect the position of the vessel on the microscope stage.

13. Apparatus according to claim 11, characterized by the fact that the capillary (4) of the injection unit is adjustable according to its angle Θ relative to the optical axis of the microscope (1).

14. Apparatus according to claim 11, characterized by the fact that the injection means include drive means for imparting an axial movement of the capillary (4).

15. Apparatus according to claim 11, characterized by the fact that the computer (11) is connected to the control unit (16) of the drive means of the object stage (5) and the micromanipulator (3).

16. Apparatus according to claim 11, characterized by the fact that the computer program is configured for storing several sets of image coordinates determined by the graphic means and corresponding sets of object coordinates computed therefrom via the graphic means.

## Revendications

1. Procédé de micro-injection dans des cellules et d'aspiration d'éléments contenus dans des cellules ou de cellules contenues dans des cultures cellulaires, un capillaire (4) étant positionné par rapport aux cellules placées sous un microscope (1) et observées au moyen d'une caméra de télévision (2), caractérisé en ce que
- un repère (30) est superposé à l'image télévisée,
- ledit repère (30), est positionné sur les cellules cibles (31) de l'image télévisée (10) par un déplacement par rapport à cette dernière, et que les coordonnées-image des cellules ainsi marquées sont enregistrées dans un ordinateur (11),
- les coordonnées-image sont converties par ledit ordinateur en coordonnées-objet du système de positionnement (19, 20, 21) du capillaire (4) ou du support de cellules (platine 5),
- l'on procède à l'injection ou à l'aspiration au niveau des cellules retenues (31), en positionnant le capillaire (4) à l'aide de l'ordinateur sur les coordonnées-objet, obtenues par transformation mathématique, de chaque cellule retenue (31).

2. Procédé selon la revendication 1, caractérisé en ce que le mouvement de perforation exécuté par le capillaire (4) dans le sens de son axe longitudinal s'accompagne d'un mouvement complémentaire du système de positionnement (19 - 21) du capillaire (4) ou du support de cellules (platine 5) selon deux coordonnées, mouvement qui suit l'angle d'incidence Θ du capillaire (4).

3. Procédé selon la revendication 1, caractérisé en ce que la zone reproduite de la préparation est subdivisée en plusieurs segments (33, 34) consécutifs de taille égale et que les bords desdits segments et l'identification de chaque segment sont superposés à l'image télévisée.

4. Procédé selon la revendication 1, caractérisé en ce que les cellules sont contenues dans un récipient (18) portant des marques de référence (35) et que le système des coordonnéesimage est mis en coïncidence avec lesdites marques de référence visibles dans l'image télévisée par un calibrage préalablement exécuté.

5. Procédé selon la revendication 1, caractérisé en ce que les coordonnées des cellules traitées (31), associées à un numéro d'identification, ainsi que les coordonnées des marques de référence (35) sont stockées en permanence une fois l'injection ou l'aspiration terminée.

6. Procédé selon la revendication 1, caractérisé en ce que l'injection ou l'aspiration commandée par ordinateur est précédée d'un test manuel au cours duquel les coordonnées de la pointe du capillaire (41) et celles de la cellule (31) sont mises en coïncidence, puis enregistrées par l'ordinateur.

7. Procédé selon la revendication 1, caractérisé en ce que l'ordinateur (11) commande la durée de l'injection et la quantité injectée ou aspirée en fonction de valeurs prédéterminées.

8. Procédé selon la revendication 1, caractérisé en ce que le capillaire (4) peut exécuter le mouvement de perforation de la cellule (31) à une vitesse réglable.

9. Procédé selon la revendication 1, caractérisé en ce que la variation de la pression d'injection dans le temps est commandée par l'ordinateur (11) en coordination avec les phases du mouvement exécuté par le capillaire (4).

10. Procédé selon la revendication 1, caractérisé en ce que les cellules à soumettre à l'injection ou à l'aspiration sont sélectionnées par un système d'analyse d'images.

11. Dispositif pour la mise en pratique du procédé selon l'une quelconque des revendications 1 à 10, caractérisé en ce qu' il comprend
- un microscope (1) muni d'une platine porte-objet (5) qui est mobile dans deux directions au moins,
- un équipement TV (2) servant à la reproduction de l'image microscopique sur un écran (10),
- un ordinateur (11) pouvant fonctionner en mode graphique et doté d'une mémoire connectée (12) et d'un dispositif (13) de superposition des symboles graphiques à l'écran (10),
- un dispositif (14) de positionnement d'un repère graphique sur l'écran (10),
- un micromanipulateur (3), sur lequel est montée une unité d'injection (4, 28) ou d'aspiration, susceptible d'être positionné par l'ordinateur (11) dans une direction au moins,
- d'un récipient porte-échantillon (18) recevant les cellules (31) à soumettre à l'injection ou à l'aspiration
- et un programme de calcul servant à convertir les coordonnées-image déterminées par l'intermédiaire de l'équipement graphique (13, 14) en coordonnées-objet, ainsi qu'à assurer la commande du micromanipulateur (3) ou de la platine porte-objet (5) par l'ordinateur (11).

12. Dispositif selon la revendication 11, caractérisé en ce que les récipients porte-échantillon (18) portent un marquage visible (35) servant à l'identification de la position du récipient sur la platine microscopique.

13. Dispositif selon la revendication 11, caractérisé en ce le capillaire (4) de l'unité d'injection occupe une position angulaire Θ qui est réglable par rapport à l'axe optique (36) du microscope (1).

14. Dispositif selon la revendication 11, caractérisé en ce l'unité d'injection est équipée d'un mécanisme d'entraînement agissant dans le sens axial du capillaire (4).

15. Dispositif selon la revendication 11, caractérisé en ce l'ordinateur (11) est relié aux commandes (16) des mécanismes d'entraînement de la platine porte-objet (5) ou du micromanipulateur (3).

16. Dispositif selon la revendication 11, caractérisé en ce que le programme de calcul prévoit la mise en mémoire de plusieurs ensembles de coordonnées-image déterminés par l'intermédiaire de l'équipement graphique et de plusieurs ensembles de coordonnées-objet calculés sur cette base.
